(19)

Europäisches
Patentamt
European
Patent Office
Office européen
des brevets

(11) **EP 2 859 904 A1**

(12) **EUROPEAN PATENT APPLICATION**
published in accordance with Art. 153(4) EPC

(43) Date of publication:
**15.04.2015 Bulletin 2015/16**

(21) Application number: **13804236.1**

(22) Date of filing: **11.06.2013**

(51) Int Cl.:
*A61M 1/18* (2006.01)      *B01D 69/02* (2006.01)
*B01D 69/08* (2006.01)      *B01D 69/12* (2006.01)
*B01D 71/08* (2006.01)      *B01D 71/38* (2006.01)
*B01D 71/44* (2006.01)      *B01D 71/68* (2006.01)
*D01D 5/24* (2006.01)      *A61K 35/14* (2015.01)
*A61P 7/08* (2006.01)

(86) International application number:
**PCT/JP2013/066053**

(87) International publication number:
**WO 2013/187396 (19.12.2013 Gazette 2013/51)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **11.06.2012   JP 2012132275**

(71) Applicant: **Asahi Kasei Medical Co., Ltd.
Tokyo 101-8101 (JP)**

(72) Inventors:
• **ICHI, Takahiro
Tokyo 101-8101 (JP)**

• **KOIZUMI, Toshinori
Tokyo 101-8101 (JP)**
• **HATTORI, Makiko
Tokyo 101-8101 (JP)**
• **SAKABE, Terumi
Tokyo 101-8101 (JP)**

(74) Representative: **Von Kreisler Selting Werner -
Partnerschaft
von Patentanwälten und Rechtsanwälten mbB
Deichmannhaus am Dom
Bahnhofsvorplatz 1
50667 Köln (DE)**

(54) **SEPARATION MEMBRANE FOR BLOOD TREATMENT AND BLOOD TREATMENT DEVICE HAVING SAME MEMBRANE INCORPORATED THEREIN**

(57)      An object of the present invention is to provide a separation membrane for blood treatment excellent in blood compatibility and reduced in elution of nitric acid ion from an inner surface even if radiation sterilization is applied in a dry state and to provide a blood treatment device having the membrane incorporated therein.

The present invention provides a separation membrane for blood treatment containing at least a polysulfone resin and polyvinylpyrrolidone, wherein a proportion of intermediate water present in water in a functional separation surface of the separation membrane is 40% or more at the time when the content of water reaches a saturation point by impregnating a dry-state separation membrane with water, a water content of the separation membrane is 10% or less, and the separation membrane is subjected to radiation sterilization.

EP 2 859 904 A1

**Description**

Technical Field

The present invention relates to a separation membrane for blood treatment and a blood treatment device having same membrane incorporated therein.

Related Art

In extracorporeal circulation therapy, a hollow fiber membrane-based blood treatment device using a selective separation membrane has been widely used. Such a hollow fiber membrane-based blood treatment device is, for example, used in hemodialysis employed as a maintenance therapy for patients with chronic renal failure; in continuous hemofiltration, continuous hemodiafiltration, continuous hemodialysis employed as an acute blood purification therapy for patients with severe pathologic conditions such as acute renal failure and sepsis; and for supplying oxygen to blood during open heart surgery or plasma separation.

In these uses, a hollow fiber membrane is required to have excellent mechanical strength and chemical stability, permeability that is easily controlled, little amount of effluent, less interaction with living-body components, and safety to a living body.

Recently, in view of mechanical strength, chemical stability and permeability control, a selective separation membrane made of a polysulfone resin has been rapidly and widely in use. Since the polysulfone resin is a hydrophobic polymer, if the polysulfone resin is used as it is, the resultant membrane will have significantly low surface hydrophilicity and poor blood compatibility, interact with blood components and be likely to cause blood coagulation and the like. In addition, protein components adsorb to the membrane, permeability is likely to reduce.

Then, to overcome these drawbacks, an attempt has been made to add a hydrophilic polymer, such as polyvinylpyrrolidone (PVP), polyvinyl alcohol and polyethylene glycol, other than a hydrophobic polymer such as a polysulfone resin in order to provide blood compatibility to a selective separation membrane. For example, a method for enhancing blood compatibility by increasing hydrophilicity of a membrane by forming a membrane by using a spinning dope for membrane formation in which a hydrophobic polymer and a hydrophilic polymer are blended; a method of providing blood compatibility by applying a hydrophilic polymer by forming a membrane by using a hollow-internal fluid containing a hydrophilic polymer in accordance with in a wet-dry membrane formation process, followed by drying, or by bringing a membrane formed into contact with a solution containing the hydrophilic polymer, followed by drying, are known.

Patent Literatures 1 to 3 disclose the amount of a hydrophilic polymer present in a membrane surface and do not disclose the state of a hydrophilic polymer present in the surface.

In the meantime, in the extracorporeal circulation therapy, blood is directly in contact with a selective separation membrane of a blood treatment device. Thus, it is necessary that the selective separation membrane is sterilized before use.

In the sterilization processing, e.g., ethylene oxide gas, high-pressure steam and radioactive rays are used. However, sterilization with ethylene oxide gas and sterilization with high-pressure steam have problems such as allergy caused by a residual gas, and processing capacity of a sterilization devices, and heat deformation of a material. Because of these, radiation sterilization with e.g., gamma-ray and an electron beam has been mainly used.

On the other hand, handling and freezing during storage in cold regions are also problems. Because of these problems, a dry-type blood treatment device has been mainly used; however, in a radiation sterilization step, generated radicals cause crosslinking reaction and decomposition, and further oxidation degradation of a hydrophilic polymer. As a result, a membrane material denatures, and thus blood compatibility reduces and an effluent increases.

As a method for preventing such deterioration of a membrane material, which is to be applied to the case where a blood treatment device is not a dry product, Patent Literature 4 discloses a method of preventing oxidation degradation by charging a membrane module with an antioxidant solution and subjecting the membrane module to gamma ray sterilization; and Patent Literature 5 discloses a method of suppressing oxidation of a filling fluid by charging a membrane module with a pH buffer and an aqueous alkaline solution and sterilizing the module.

In contrast, in the case where a blood treatment device is a dry product, Patent Literature 6 discloses a method of controlling the oxygen concentration during sterilization to be 0.001% or more and 0.1% or less. However, in the technique of Patent Literature 6, a packaging bag must be sterilized by substituting the inner atmosphere with an inert gas or it is necessary to enclose a deoxidant in a packaging bag and subject sterilization after a lapse of a predetermined time. In addition, if an oxygen concentration just exceeds only by 0.1%, sufficient blood compatibility cannot be obtained by radiation sterilization.

In Non Patent Literature 1, biocompatibility in connection with the state of water contained in a polymer material such as poly (2-methoxyethylacrylate) (PMEA) is reported under the title of "The Roles of organized water molecules in the biointerface".

According to Non Patent Literature 1, if a general polymer material is impregnated with water, water in the polymer is present in the following two states: (1) "non-freezing water", which is the state of water not frozen even at -100°C due to strong interaction with a polymer, (2) "free water", which is the state of water thawing at 0°C but having weak interaction with a polymer or non-freezing water; however, in a polymer excellent in biocompatibility, another state of water: (3) "intermediate water" is present, which is water frozen at a lower temperature than 0°C during a temperature increasing process and having an intermediate interaction with a polymer or non-freezing water. However, a polymer inferior in biocompatibility, no "intermediate water" is present.

Furthermore, Non Patent Literature 1 discloses the result suggesting a close relationship between the presence of "intermediate water" in the water of a polymer and excellent biocompatibility exhibited by the polymer.

The mechanism of "intermediate water" for influencing the biocompatibility of a polymer is considered as follows.

The "free water", since it freely exchanges with bulk water, i.e., general water having no interaction with a polymer, is not involved in coating the surface of a polymer material; whereas "non-freezing water" is present so as to coat the surface of a polymer material due to strong interaction with a polymer material. However, "non-freezing water" mutually acts with a hydration shell itself of a living-body component such as a protein, which is stabilized by the formation of a hydration shell in blood, to destroy the hydration-shell structure. If the hydration shell is destroyed, a living-body component adsorbs to the surface of a polymer material. Therefore, if a general polymer material only having "free water" and "non-freezing water" is used, a living-body component recognizes the surface of a polymer material as a foreign substance and an immune reaction is initiated.

The "intermediate water" binds to a polymer material by the interaction with "non-freezing water" and covers the surface of "non-freezing water"; however, the "intermediate water" does not have a specific hydrogen binding structure that destroys the hydration shell of a living-body component. Thus, a living-body component fails to recognize the surface of a polymer material as a foreign substance. Therefore, it is presumed that a polymer material having "intermediate water" is excellent in blood compatibility.

Furthermore, Non Patent Literature 2 reports that experimental animals are affected by nitrate administration and that a nitrate is almost completely excreted from urea, although causal relation between nitrate intake and health damage is not clearly elucidated. In consideration of disclosure of Non Patent Literature 2, intake of a nitrate should be reduced in patients with renal failure, and elution of nitric acid ion also in the separation membrane for blood treatment and a blood treatment device seems to be preferably reduced.

Citation List

Patent Literature

Patent Literature 1: Japanese Patent 3551971
Patent Literature 2: Japanese Patent Laid-Open No. 2002-212333
Patent Literature 3: Japanese Patent Laid-Open No. 6-296686
Patent Literature 4: Japanese Patent Laid-Open No. 4-338223
Patent Literature 5: Japanese Patent Laid-Open No. 7-194949
Patent Literature 6: International publication No. 2006/016575

Non Patent Literature

Non Patent Literature 1: Ken Tanaka, The Roles of organized water molecules in the biointerface, Sakigake Live 2004, Nano-technology field Joint Research Debrief Session, Summary of lecture in the section of "Systemization and Function" (investigation period: 2001-2004), 2005, p. 24-33, URL:<http://jstore.jst.go.jp/PDFView.html?type=research&i d=3090&property=researchReportPdfList&index=0>, [access date: November 16, 2011]
Non Patent Document 2: Soft Drink Evaluation Document, nitrate nitrogen/nitrite nitrogen (draft), Food Safety Commission, chemical substance/contaminant specialist research meeting, May, 2012, URL:<http://www.fsc.go.jp/ikenbosyu/pc1_ko_seiryo_nitrogen_240524.pdf>, [access date: May 2, 2013]

**EP 2 859 904 A1**

Summary of Invention

Technical Problem

An object of the present invention is to provide a separation membrane for blood treatment excellent in blood compatibility even if radiation sterilization is applied in a dry state and reduced in amount of nitric acid ion eluted from an inner surface and a blood treatment device having the membrane incorporated therein.

Solution to Problem

The present inventors conducted intensive studies with the view to solving the aforementioned problems. As a result, they found that it is important to maintain a hydrophilic polymer in a separation membrane at an appropriate state in order to provide excellent blood compatibility to a separation membrane for blood treatment and to reduce elution of nitric acid ion from an inner surface.

More specifically, they intensively studied on the surface of a practicable separation membrane in contact with blood, in view of blood compatibility. As a result, they found that if intermediate water can be allowed to exist in the functional separation surface of the separation membrane, provided that a proportion of intermediate water is 40% or more at the time when the content of water reaches a saturation point by impregnating a dry-state separation membrane with water, not only sufficient blood compatibility can be exhibited but also the amount of nitric acid ion eluted from the inner surface can be reduced. Based on the findings, the present invention was accomplished.

More specifically, the present invention is as follows.

[1] A separation membrane for blood treatment containing at least a polysulfone resin and polyvinylpyrrolidone, wherein

the proportion of intermediate water present in water in a functional separation surface of the separation membrane is 40% or more at the time when the content of water reaches a saturation point by impregnating a dry-state separation membrane with water,

a water content of the separation membrane is 10% or less, and the separation membrane is subjected to radiation sterilization.

[2] The separation membrane for blood treatment according to [1], wherein an amount of nitric acid ion eluted from an inner surface is 0.2 ppm or less.

[3] The separation membrane for blood treatment according to [1] or [2], having a polymer having a function of suppressing radiation deterioration of polyvinylpyrrolidone at least in the functional separation surface of the separation membrane.

[4] The separation membrane for blood treatment according to any one of [1] to [3], wherein the radiation sterilization is performed at an irradiation dose of 15 to 50 kGy.

[5] The separation membrane for blood treatment according to [3] or [4], wherein the polymer having a function of suppressing radiation deterioration of polyvinylpyrrolidone is at least one selected from the group consisting of polyhydroxyalkyl methacrylate and a sodium salt of a polysaccharide.

[6] A blood treatment device containing the separation membrane for blood treatment according to any one of [1] to [5] incorporated therein.

[7] A method for producing a separation membrane for blood treatment containing at least a polysulfone resin and polyvinylpyrrolidone, comprising the steps of:

forming a separation membrane containing at least a polysulfone resin and polyvinylpyrrolidone, drying the never dry membrane with heated steam up to a water content of the separation membrane of 10% or less, and

subjecting the separation membrane to radiation sterilization.

[8] A method for producing a separation membrane for blood treatment containing at least a polysulfone resin and polyvinylpyrrolidone, comprising the step of:

forming a separation membrane containing at least a polysulfone resin and polyvinylpyrrolidone, drying the separation membrane up to a water content of 10% or less, and

subjecting the separation membrane, which has a polymer having a function of suppressing radiation deterioration of polyvinylpyrrolidone in the functional separation surface of the separation membrane, to radiation sterilization.

4

[9] The production method according to [8], comprising a step of coating the functional separation surface of the separation membrane with the polymer by ejecting a spinning dope for membrane formation containing at least the polysulfone resin and polyvinylpyrrolidone and a hollow-internal fluid containing the polymer, followed by spinning.
[10] The production method according to [8], comprising a step of coating the functional separation surface of the separation membrane with the polymer by injecting a coating liquid containing the polymer into a separation membrane containing at least a polysulfone resin and polyvinylpyrrolidone.

Advantageous Effects of Invention

The separation membrane for blood treatment and the blood treatment device having the membrane incorporated therein according to the present invention exhibit satisfactory blood compatibility and an effect of reducing the amount of nitric acid ion eluted from the inner surface even if they are subjected to radiation sterilization in a dry state.

Brief Description of Drawings

[Figure 1] Figure 1 shows how to set a sample for infrared spectroscopy (IR) for calculating the proportion of intermediate water present in water in the functional separation surface of the separation membrane at the time when the content of water reaches a saturation point by impregnating a dry-state separation membrane with water.
[Figure 2] Figure 2 shows an example of experiment spectrum matrix A.

Description of Embodiments

Now, an embodiment for carrying out the invention (hereinafter, refers to "the embodiment") will be more specifically described below. Note that the present invention is not limited to the embodiment described below and modified in various ways within the range and carried out.
The separation membrane for blood treatment of the embodiment (hereinafter, sometimes simply referred to as the "separation membrane") is a separation membrane containing at least a polysulfone resin and polyvinylpyrrolidone, in which the proportion of intermediate water present in water in the functional separation surface of the separation membrane is 40% or more at the time when the content of water reaches a saturation point by impregnating a dry-state separation membrane with water and the water content of the separation membrane is 10% or less. Furthermore, the separation membrane is sterilized with radiation.
The separation membrane of the embodiment contains a polysulfone resin as a main component and further contains polyvinylpyrrolidone as a hydrophilic polymer, providing blood compatibility to the separation membrane. In the embodiment, a separation membrane containing at least a polysulfone resin and polyvinylpyrrolidone and containing the polysulfone resin as a main component can be obtained by controlling the concentrations of the polysulfone resin and polyvinylpyrrolidone in a spinning dope for membrane formation to fall within the concentration ranges as described below. Herein, "at least" means that a polysulfone resin and polyvinylpyrrolidone serving as structural components are contained as essential components in the separation membrane and the presence of other structural components is acceptable.

<Polysulfone resin>

The polysulfone resin refers to a synthetic polymer containing a sulfonyl group ($-SO_2-$) and excellent in heat resistance and chemical resistance.
Examples of the polysulfone resin include polyphenylene sulfone, polysulfone, polyarylether sulfone, polyethersulfone and copolymers of these.
The polysulfone resins may be used singly or as a mixture of two or more.
Particularly, to control fractionation, a polysulfone polymer represented by the following formula (1) or the following formula (2) is preferable

$$(-Ar-SO_2-Ar-O-Ar-C(CH_3)_2-Ar-O-)_n \qquad (1)$$

$$(-Ar-SO_2-Ar-O-)_n \qquad (2)$$

where Ar represents a benzene ring; and n represents the number of monomer unit repeats. The polysulfone represented by formula (1) is commercially available, for example, from Solvey under the name of UDEL (trade mark) and from BASF under the name of "Ultrason (trade mark)". Whereas, the polyethersulfone represented by formula (2) is commercially available, for example, from Sumitomo Chemical Co., Ltd., under the name of "SUMIKAEXCEL (trade mark)". Since several types of polysulfone compounds varied in polymerization degree are present, they can be appropriately used.

<Polyvinylpyrrolidone>

Polyvinylpyrrolidone refers to a water-soluble hydrophilic polymer obtained by vinyl polymerization of N-vinyl pyrrolidone and is widely used as a material for a hollow fiber membrane for a hydrophilizing agent and a pore-forming agent.

As the polyvinylpyrrolidone, several types of compounds different in molecular weight are commercially available from BASF under the name of "LUVITEC (trade mark)" and these can be appropriately used.

Polyvinylpyrrolidone compounds may be used singly or as a mixture of two or more.

<The proportion of intermediate water present in water in the functional separation surface of the separation membrane at the time when the content of water reaches a saturation point by impregnating a dry-state separation membrane with water>

The separation membrane of the embodiment is a membrane having a proportion of intermediate water present in water in the functional separation surface of the separation membrane (hereinafter, sometimes simply referred to as "ratio of intermediate water") of 40% or more at the time when the content of water reaches a saturation point by impregnating a dry-state separation membrane with water.

In the separation membrane of the embodiment, in view of blood compatibility, a never dry membrane is dried or a hydrophilic polymer is provided in addition to use of a polysulfone resin. Furthermore, the proportion of intermediate water present in the functional separation surface of practicable separation membrane, near the site of the membrane surface in contact with blood, is specified to fall within a predetermined range. Owing to these, the separation membrane exhibits satisfactory blood compatibility and an effect of reducing the amount of nitric acid ion eluted from the inner surface, even if radiation sterilization is applied in a dry state.

In the embodiment, the proportion of intermediate water in a process of impregnating a dry-state separation membrane with water is computationally obtained by measuring the attenuated total reflection infrared absorption (ATR-IR) in the functional separation surface of a separation membrane and analyzing the absorption with time.

In the embodiment, "the functional separation surface of the separation membrane" refers to the region corresponding to the thickness of a contact surface to blood detected by ATR-IR. More specifically, the functional separation surface of the separation membrane refers to a detectable region by ATR-IR measurement, and usually refers to a region corresponding to the thickness of 1 $\mu$m or less from the membrane surface.

In the embodiment, "at the time when the content of water reaches a saturation point by impregnating a dry-state separation membrane with water" refers to a time point at which water is not absorbed anymore in the process of impregnating a dry-state separation membrane with water, that is, a time point at which the intensity of the peak (at 3000 to 3700 $cm^{-1}$) derived from a hydroxy group no longer increases relative to the intensity of the peak (near 1485 $cm^{-1}$) derived from a benzene ring of a polysulfone resin, measured in ATR-IR measurement. In the embodiment, "dry state" refers to the state where the water content of the separation membrane reaches the equilibrium moisture regain, as illustrated by an example in Examples. "Process of impregnating a dry-state separation membrane with water" refers to the process during which a dry-state separation membrane is impregnated with water, as illustrated by an example in Examples.

In the embodiment, in order to provide biocompatibility to a separation membrane and reduce elution of nitric acid ion from the inner surface thereof, polyvinylpyrrolidone must have a property of maintaining intermediate water such that the proportion of intermediate water present in water in the functional separation surface of the separation membrane becomes 40% or more at the time when the content of water reaches a saturation point by impregnating a dry-state membrane with water even after radiation sterilization.

For example, if a radiation ray is applied to a polymer, radicals are generated on the polymer and the polymer is attacked by oxygen radicals. Depending upon the radical generation site or attacking site, breakage and crosslinking of the main chain of the polymer and cleavage of a side chain may likely occur. If the main chain of polyvinylpyrrolidone of a separation membrane for blood purification is broken and the molecular weight of polyvinylpyrrolidone is reduced, polyvinylpyrrolidone is likely to elute. If crosslinking and cleavage of a side chain occur, the hydrophilicity of the separation membrane due to polyvinylpyrrolidone reduces.

As a result, an effect of providing biocompatibility to a separation membrane cannot be sufficiently exerted and the amount of nitric acid ion eluted from the inner surface increases.

Since the radical reaction is complicated, various types of final products are resulted; however, the state of polyvinylpyrrolidone, even if it is slightly changed, is presumed to have an effect on biocompatibility of a separation membrane. However, a change of the state of polyvinylpyrrolidone in connection with blood compatibility was not detected by a conventional spectroscopic method at the level (50 kGy or less) of radiation sterilization generally applied to medical devices.

In the embodiment, it has been elucidated that a change of the state of polyvinylpyrrolidone present in separation-membrane surface in connection with blood compatibility can be detected as a change in the proportion of intermediate water present in water in the functional separation surface of the separation membrane by time-resolved ATR-IR measurement and analysis for the measurement during a water impregnation process. To be more specific, as the proportion of intermediate water reduces, the state of polyvinylpyrrolidone changes toward blood incompatibility; at the same time, the amount of nitric acid ion eluted from the inner surface increases.

The present inventors further conducted studies. As a result, it was elucidated that, in order to provide biocompatibility to a separation membrane and reduce elution of nitric acid ion from the inner surface, it is necessary that the proportion of intermediate water present in water in the functional separation surface of the separation membrane becomes 40% or more at the time point when the content of water reaches a saturation point in the process of impregnating a dry-state separation membrane with water. In view of blood compatibility, the proportion of intermediate water is preferably 50% or more.

In the embodiment, in order to control the proportion of intermediate water to be 40% or more, the separation membrane to be subjected to radiation sterilization is prepared by drying, for example, a wet-state membrane obtained after spinning, in other words, a never dry membrane, by heated steam, or the functional separation surface of the separation membrane is coated with a polymer having a function of suppressing radiation deterioration of polyvinylpyrrolidone. The reason why the proportion of intermediate water was successfully controlled to be 40% or more by these methods has not yet been elucidated; however, the following reasons are presumed. It is considered that, by performing drying and structural fixation in the conditions as if water is present around the membrane in view of water and a hydroxy group of a polymer during the drying time, the orientation and tertiary structure of polyvinylpyrrolidone can be maintained close to those obtained in the presence of water, and intermediate water becomes easily retained, suppressing decomposition of a side chain caused by transfer of radicals generated. As a result, the polyvinylpyrrolidone present in the functional separation surface of the separation membrane is suppressed from being excessively decomposed. For the reason, the effect of providing biocompatibility is not reduced, and adsorption of protein to a separation membrane and activation by the membrane surface can be suppressed to obtain a separation membrane having high blood compatibility; at the same time, the amount of nitric acid ion eluted from the inner surface can be reduced.

In the embodiment, in the drying method by heated steam, the separation membrane is directly rolled up while keeping a wet state, bundled, wrapped in, e.g., a PE film, placed in a drying chamber and dried by introducing heated steam in the chamber. The pressure to be applied at this time may be normal pressure or vacuum. The temperature of heated steam is preferably inversion temperature (temperature at which the same evaporation rate is obtained irrelevant with humidity) or more and 200°C or less in order to save drying time and suppress thermal decomposition.

<Polymer having a function of suppressing deterioration of polyvinylpyrrolidone with irradiation>

In the embodiment, a polymer having a function of suppressing deterioration of polyvinylpyrrolidone with irradiation (hereinafter, sometimes simply referred to as the "polymer") is not particularly limited as long as the polymer can be applied to the surface of polyvinylpyrrolidone and suppress decomposition and crosslinkage of polyvinylpyrrolidone caused by irradiation at the time of radiation sterilization. To protect polyvinylpyrrolidone in the functional separation surface of the separation membrane by coating, the polymer needs to be dissolved in water, a spinning dope, a hollow-internal fluid or a coating liquid and has a hydroxy group for unknown reasons.

The polymer is preferably insoluble or hardly-soluble in water in consideration of elution into a priming processing liquid or preferably causes no trouble even if it elutes. As the polymer insoluble or hardly-soluble in water, polyhydroxyalkyl methacrylate is favorably used. In order to coat the inner surface with a polymer, the polymer has a weight average molecular weight of preferably 10000 or more, more preferably 100000 or more and further preferably 300000 or more. The solubility of such a polymer at 20°C in water (100 g) is preferably less than 1 g, more preferably 0.8 g or less and further preferably 0.5 g or less.

As the polymer dissolved in water, a sodium salt of a polysaccharide is favorably used; however, since it is more preferable that the polymer is discharged by priming, a polymer having a viscosity (viscosity of a 0.1% aqueous solution of the polymer) of 100 mPa·s or less at 25°C is favorably used. Since a sodium salt of a polysaccharide is extracted from a natural substance and purified, if the viscosity is high, the sodium salt may be used after it is decomposed by acid hydrolysis.

The polymers may be used singly or as a mixture of two or more.

In the embodiment, the weight average molecular weight of the polymer can be measured, for example, by gel permeation chromatography (GPC) as described in Examples below.

Furthermore, in the embodiment, the viscosity (in a 0.1% aqueous solution) of the polymer at 25°C can be measured, for example, by a viscometer as described in Examples below.

Examples of the polymer include polyhydroxyalkyl methacrylates such as polyhydroxyethyl methacrylate, polyhydroxypropyl methacrylate and polyhydroxybutyl methacrylate; and sodium salts of polysaccharides such as sodium alginate, sodium hyaluronate and heparin sodium. The polyhydroxyalkyl methacrylate is a synthetic polymer obtained by polymerizing or copolymerizing a hydroxyalkyl methacrylate as a monomer unit and having a hydroxy group at a side chain.

The polymer is desirably added in a large amount in order to improve an effect of protecting polyvinylpyrrolidone in a separation membrane against sterilization; however, the polymer is desirably added in a small amount in view of control of the membrane separation properties such as water permeability and mechanics physical properties such as strong extensibility. In considering these two points with each other, the amount of polymer to be added must be determined appropriately.

The amount of the polymer present in the functional separation surface of the separation membrane is preferably 0.9 times to 5 times, and more preferably once to three times as large as the amount of polyvinylpyrrolidone detected in the functional separation surface of the separation membrane, in view of protection effect on polyvinylpyrrolidone, control of the membrane separation properties such as permeability and mechanics physical properties such as strong extensibility.

In the embodiment, the amount of the polymer present in the functional separation surface of the separation membrane can be measured by the method described in Examples below.

In the embodiment, as a method for attaching the polymer as a separation membrane component to a separation membrane, a spinning method in which the polymer is added and dissolved in a spinning dope for forming a separation membrane and subjected to spinning, a spinning method in which the polymer is added and dissolved in a hollow-internal fluid for forming a separation membrane and subjected to spinning, and a coating method in which a coating liquid in which the polymer is dissolved is applied onto a separation membrane are favorably used.

Of these methods, in view of mechanical properties of the separation membrane, the spinning method in which the polymer is added and dissolved in a hollow-internal fluid for forming a separation membrane or the coating method is simple and carried out in a low amount of polymer.

For example, in the spinning method by adding and dissolving the polymer in a hollow-internal fluid for forming a separation membrane, polyvinylpyrrolidone present in the functional separation surface of the separation membrane can be coated with the polymer by ejecting the hollow-internal fluid dissolving the polymer and a spinning dope for forming a membrane containing a polysulfone resin, polyvinylpyrrolidone and a solvent, simultaneously from a tube-in-orifice type spinneret.

In the coating method for applying a coating liquid onto a separation membrane, preferably after the separation membrane is formed and incorporated into a blood treatment device and assembled, the separation membrane can be coated by supplying a coating liquid prepared by dissolving the polymer and allowing the liquid into contact with the functional separation surface of the separation membrane.

<Water content>

In the embodiment, a separation membrane for blood treatment having excellent blood compatibility and a reduced amount of nitric acid ion eluted from an inner surface even if radiation sterilization is applied in a dry state and a blood treatment device having the membrane incorporated therein are provided. In the embodiment, the "dry state" refers to the state of a separation membrane having a water content of 10% or less. Also in view of generation of e.g., microorganisms and mold during storage, it is preferable that the water content of the separation membrane is 10% or less. If the water content of the separation membrane exceeds 10%, e.g., dew condensation may often occur during storage and is not preferable in view of appearance. In addition, mass of the separation membrane increases. This is not suitable to transport the membranes collectively in a facility.

In the embodiment, the water content of the separation membrane can be controlled to be 10% or less by drying the separation membrane obtained by spinning, with heated steam. To the separation membrane coated with the polymer capable of suppressing deterioration of polyvinylpyrrolidone by irradiation, can be dried by a customary method using e.g., hot air to control the water content of the separation membrane to be 10% or less.

In the embodiment, the water content of the separation membrane can be measured by the method described in Examples below.

Furthermore, in the embodiment, it is possible to obtain a separation membrane for blood treatment, which contains at least a polysulfone resin and polyvinylpyrrolidone, and is excellent in biocompatibility and reduced in the amount of nitric acid ion eluted from the inner surface, (in the separation membrane, the proportion of intermediate water present in water in the functional separation surface of the separation membrane is 40% or more at the time when the content of water reaches a saturation point by impregnating a dry-state separation membrane with water) by subjecting the never dry membrane to a dry process with heated steam and thereafter subjecting the membrane (in a dry state:

water content of 10% or less) to radiation sterilization, or by coating the functional separation surface of the separation membrane with a polymer having a function of suppressing radiation deterioration of polyvinylpyrrolidone and subjecting the membrane (in a dry state: water content of 10% or less) to radiation sterilization.

<Blood treatment device>

The blood treatment device of the embodiment, which is a blood treatment device having the separation membrane of the embodiment incorporated therein, is used for an extracorporeal circulation system blood purification therapy such as hemodialysis, hemofiltration, hemodiafiltration, blood component fractionation, supplying oxygen and the plasma separation.

If blood treatment is performed by the blood treatment device, in which the separation membrane of the embodiment (although the separation membrane is subjected to radiation sterilization in a dry state) is incorporated therein, since the separation membrane satisfies the proportion of intermediate water present in water in the functional separation surface of the separation membrane of 40% or more at the time when the content of water reaches a saturation point by impregnating a dry-state separation membrane with water, the treatment can be performed with satisfactory blood compatibility and in a reduced amount of nitric acid ion eluted from the inner surface.

The blood treatment device is preferably used as e.g., a hemodialyzer, a hemofilter and a hemodiafilter, and more preferably used in continuous therapy , such as a continuous hemodialyzer, a continuous hemofilter and a continuous hemodiafilter. Depending upon the therapy, the specifications such as dimension and fractionation performance of a separation membrane are determined.

It is preferable that the separation membrane to be incorporated in a blood treatment device has a hollow portion. In view of permeability, it is further preferable to have crimps.

<Method for producing separation membrane for blood treatment>

A method for producing the separation membrane for blood treatment according to the embodiment, comprises the steps of forming a separation membrane containing at least a polysulfone resin and polyvinylpyrrolidone, drying the never dry membrane with heated steam up to a water content of the separation membrane of 10% or less, and subjecting the separation membrane to radiation sterilization.

Another method for producing the separation membrane for blood treatment according to the embodiment, includes the steps of forming a separation membrane containing at least a polysulfone resin and polyvinylpyrrolidone, drying the separation membrane up to a water content of the separation membrane of 10% or less, and subjecting the separation membrane, which has a polymer having a function of suppressing radiation deterioration of the polyvinylpyrrolidone at least in the functional separation surface of the separation membrane, to radiation sterilization.

The separation membrane is formed by a customary method using a spinning dope for membrane formation at least containing a polysulfone resin and polyvinylpyrrolidone.

The spinning dope for membrane formation is prepared by dissolving a polysulfone resin and polyvinylpyrrolidone in a solvent.

Examples of such a solvent include dimethylacetamide, dimethylsulfoxide, N-methyl-2-pyrrolidone, dimethylformamide, sulfolane and dioxane.

A single solvent may be used or two or more solvents may be used as a mixture.

The concentration of a polysulfone resin in a spinning dope for membrane formation is not particularly limited as long as it falls within the concentration range in which a membrane can be formed and the obtained membrane can act as a permeable membrane; however, the concentration is preferably 5 to 35 mass% and more preferably 10 to 30 mass%. If high water permeability is attained, the concentration of a polysulfone resin is preferably low and further preferably 10 to 25 mass%.

The concentration of polyvinylpyrrolidone in a spinning dope for membrane formation is controlled such that the mixing ratio of the polyvinylpyrrolidone to a polysulfone resin is preferably 27 mass% or less, more preferably 18 to 27 mass% and further preferably 20 to 27 mass%.

If the mixing ratio of polyvinylpyrrolidone to a polysulfone resin is 27 mass% or less, the elution amount of polyvinylpyrrolidone can be reduced. Favorably, the mixing ratio is 18 mass% or more. If so, the concentration of polyvinylpyrrolidone in the functional separation surface of the separation membrane can be controlled within a preferable range. In addition, an effect of suppressing a protein adsorption can be enhanced and excellent blood compatibility is obtained.

A flat membrane and a hollow fiber membrane are formed by using a spinning dope for membrane formation in accordance with a customary method. For example, a hollow fiber membrane is formed by ejecting a spinning dope for membrane formation from the orifice of a tube-in-orifice type spinneret, in the air, simultaneously with a hollow-internal fluid containing the polymer used for coagulating the spinning dope from a tube. As the hollow-internal fluid, water or a liquid containing water as a main component, can be used; generally a solution mixture of the solvent used in a spinning dope for membrane formation and water is favorably used. For example, a 20 to 70 mass% aqueous dimethylacetamide solution is used.

The inner diameter and thickness of the hollow fiber membrane can be controlled to desired values by controlling the ejection amount of spinning dope for membrane formation and the ejection amount of hollow-internal fluid.

The inner diameter of the hollow fiber membrane for use in blood treatment may be generally 170 to 250 $\mu$m and preferably 180 to 220 $\mu$m. In view of efficiency of diffusion removal of low molecular-weight substances due to mass transfer resistance as a permeable membrane, the thickness of the hollow fiber membrane is preferably 50 $\mu$m or less. In view of strength, the thickness of the membrane is preferably 10 $\mu$m or more.

The spinning dope for membrane formation ejected from a spinneret together with the hollow-internal fluid is passed through an air gap, introduced in a coagulation bath containing water as a main component and placed in the lower portion of the spinneret, and soaked in the bath for a predetermined time to complete coagulation. At this time, a draft, which is represented by the proportion of the linear velocity of the spinning dope for membrane formation ejection and the take-over speed, is preferably 1 or less.

The air gap refers to a space between the spinneret and the coagulation bath, and coagulation of the spinning dope for membrane formation is started from the inner surface side by a poor solvent component such as water in the hollow-internal fluid simultaneously ejected from the spinneret. When coagulation is initiated, a smooth separation-membrane surface is formed and the separation membrane structure becomes stable. Therefore, the draft is preferably 1 or less and more preferably 0.95 or less.

Subsequently, the solvent remaining on the hollow fiber membrane is removed by washing with hot water, etc. and then, the separation membrane is rolled up while keeping a wet state, and bundled by controlling the length and number, wrapped in e.g., a PE film, and the separation membrane bundle is placed in a drying chamber and dried by introducing heated steam in the chamber.

Washing is performed to remove unnecessary polyvinylpyrrolidone, preferably with hot water of 60°C or more for 120 seconds or more, and more preferably with hot water of 70°C or more for 150 seconds or more.

heated steam may be introduced at normal pressure or under vacuum. The temperature of heated steam is preferably inversion temperature (temperature at which the same evaporation rate is obtained irrelevant with humidity) or more and 200°C or less in order to save drying time and suppress thermal decomposition.

Since embedding with a urethane resin is performed in a later step and radiation sterilization is performed in a dry state in the embodiment, the water content of the separation membrane is specified as 10% or less.

The hollow fiber membrane bundle obtained through the aforementioned steps is subjected to a module production step. In this step, a cylindrical container having two nozzles near both end portions of the side surface is charged with the hollow fiber membrane bundle and the both end portions are embedded with a urethane resin. Subsequently, hardened urethane portions are cut to obtain a hollow fiber membrane with open end portions. The both end portions are closed with header caps having a nozzle for introducing (discharging) liquid and a blood treatment device is fabricated.

Subsequently, the blood treatment device in which the hollow fiber separation membrane obtained by drying from a never dry state by heated steam is subjected to radiation sterilization processing. For radiation sterilization, e.g., an electron beam, gamma ray and X-ray can be used and any one of them is acceptable. The irradiation dose in the cases of an electron beam and gamma ray, usually falls within the range of 15 to 50 kGy and preferably 20 to 40 kGy. The blood treatment device is subjected to a sterilization step such as radiation sterilization to obtain a final device.

In the method for producing a hollow fiber separation membrane having the polymer in the functional separation surface of the separation membrane, including coating the separation membrane with the polymer in the embodiment, a polymer having a function of suppressing radiation deterioration of polyvinylpyrrolidone may be dissolved in the hollow-internal fluid for use in coagulation of a spinning dope in a desired concentration and then applied to polyvinylpyrrolidone. The concentration of the polymer in the hollow-internal fluid, when a synthetic polymer such as polyhydroxypropyl methacrylate and polyhydroxyethyl methacrylate is used, may fall within the range of preferably 0.005 mass% to 1 mass% and more preferably 0.01 mass% to 0.1 mass%, based on the hollow-internal fluid. In contrast, when a natural polymer such as sodium alginate and sodium hyaluronate is used, since the solubility thereof in water is higher than that of the above synthetic polymer and coating-film formation performance is high, the concentration may fall within the range of preferably 0.001 mass% to 1 mass% and more preferably 0.005 mass% to 0.1 mass% based on the hollow-internal fluid.

If the viscosity of the hollow-internal fluid is high, the molecular weight of a polymer may be controlled before use by decomposing the polymer with acid hydrolysis.

Polyvinylpyrrolidone present in the inner surface can be coated with a polymer by ejecting a hollow-internal fluid containing the polymer.

The inner diameter of the hollow fiber membrane used for blood treatment may be generally 170 to 250 $\mu$m and preferably 180 to 220 $\mu$m. In view of efficiency of diffusion removal of low molecular-weight substances due to mass transfer resistance as a permeable membrane, the thickness of the hollow fiber membrane is preferably 50 $\mu$m or less.

In view of strength, the thickness of the membrane is preferably 10 μm or more.

Subsequently, the solvent remaining on the hollow fiber membrane is removed by washing with hot water, etc. and then, the hollow fiber membrane is continuously introduced into a dryer and dried by hot air, etc. to obtain a dry hollow fiber membrane. Through the coagulation step to the dry step, the polymer present in the hollow-internal fluid is applied to the polyvinylpyrrolidone present in the functional separation surface of the separation membrane.

Washing is performed to remove unnecessary polyvinylpyrrolidone preferably with hot water of 60°C or more for 120 seconds or more and more preferably with hot water of 70°C or more for 150 seconds or more.

Since embedding with a urethane resin is performed in a later step and radiation sterilization is performed in a dry state in the embodiment, the water content of the separation membrane is specified as 10% or less.

The hollow fiber membrane obtained through the aforementioned steps is bundled by controlling the length and number so as to obtain a desired membrane area and subjected to a module production step. In this step, a cylindrical container having two nozzles near both end portions of the side surface is charged with the hollow fiber membrane and the both end portions are embedded with a urethane resin. Subsequently, a hardened urethane is cut to obtain a hollow fiber membrane with open end portions. The both end portions are closed with header caps having a nozzle for introducing (discharging) liquid and a blood treatment device is fabricated.

After the module is fabricated, the coating liquid containing the polymer is injected into a hollow membrane to coat the polyvinylpyrrolidone present in the functional separation surface of the separation membrane with the polymer. In this case, a blood treatment device is fabricated in the same manner as in the case where the membrane is formed by use of hollow-internal fluid containing the polymer except that the membrane is formed by use of hollow-internal fluid containing no polymer.

The coating liquid is not particularly limited as long as it is a solvent that does not dissolve a polysulfone but dissolve the polymer; however, water and an aqueous alcohol solution are preferred in view of safety of the step and handling in the following drying step. In view of the boiling point and toxicity, water, an aqueous ethanol solution and an aqueous isopropyl alcohol solution are favorably used. The concentration of the polymer in the coating liquid may be preferably 0.001 mass% to 1 mass% and more preferably 0.005 mass% to 0.1 mass% based on the coating liquid.

The coating liquid is injected through the header cap having a nozzle. Subsequently, excessive solution is removed by use of compressed air. Thereafter, drying may be performed under vacuum or by heat dry until a constant amount is obtained. The temperature of the heat dry is not particularly limited as long as the members of the module do not deteriorate and may be appropriately set in consideration of time required for the step.

Subsequently, the blood treatment device, obtained by coating the separation membrane with the polymer, in which the hollow fiber separation membrane having the polymer in the functional separation surface of the separation membrane is incorporated, is subjected to radiation sterilization processing. For radiation sterilization, e.g., an electron beam, gamma ray and X-ray can be used and any one of them is acceptable. The irradiation dose in the cases of an electron beam and gamma ray, usually falls within the range of 15 to 50 kGy and preferably 20 to 40 kGy. The blood treatment device is subjected to a sterilization step such as radiation sterilization to obtain a final device.

Examples

Now, the present invention will be more specifically described below by way of Examples and Comparative Examples; however, the present invention is not limited to these Examples. Note that, the measurement methods used in Examples are as follows.

[Calculation of the proportion of intermediate water present in water in the functional separation surface of the separation membrane at the time when the content of water reaches saturation point by impregnating dry-state separation membrane with water]

Calculation of a proportion of intermediate water was performed in the following procedures: (1) IR measurement, (2) determination of a time point when the content of water reaches a saturation point, and (3) data analysis by chemometrics.

(1) IR measurement

Time-resolved IR measurement was performed in the conditions shown in Table 1. The procedure of sampling was as follows. The inner surface of a hollow separation membrane was washed with distilled water at a rate of 100 mL/min per 1.5 $m^2$ for 5 minutes, and then, the outer surface of the hollow separation membrane was washed at a rate of 500 mL/min for 2 minutes. In this manner, priming was performed. After the priming, the blood treatment device was decomposed and the hollow separation membrane was sampled. The hollow separation membrane (sample) was previously freeze-dried and allowed to stand still in a constant-temperature and humidity chamber of temperature of 23°C and a humidity of 50%, for 24 hours or more. The sample that reached an equilibrium moisture regain (defined as the "dry state") was subjected to measurement.

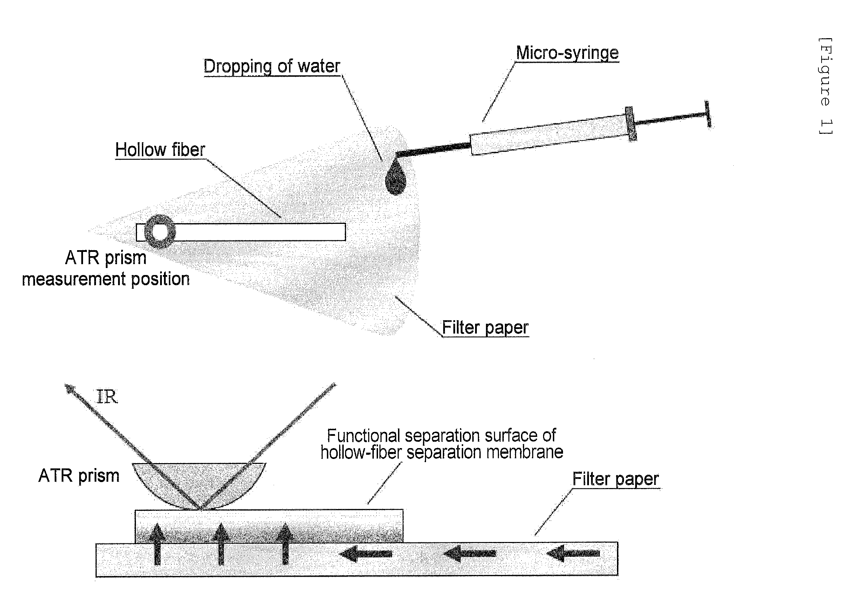

1) KIRIYAMA filter paper (No. 5C, the size of particle that can be trapped: 1 $\mu$m) of 40 mm$\varphi$ in diameter was cut into 1/8 pieces having a fan shape.
2) A sample was cut by a razor and placed on the filter paper such that the functional separation surface of the hollow-fiber separation membrane faced up, as shown in Figure 1.
3) An ATR prism was brought into contact with the functional separation surface of the hollow-fiber separation membrane and distilled water (13 to 15 $\mu$L) was added dropwise at the circular-arc edge of the fan-shape filter paper by means of a micro-syringe. Time-resolved IR measurement was performed until the time point when water permeated to the hollow fiber and the OH peak was saturated or later.

[Table 1]
Measurement conditions of time-resolved IR measurement

| | |
|---|---|
| Apparatus: | FTS-575C/UMA500 (manufactured by Varian) |
| Measurement method: | ATR method (Ge prism : 30 degrees) |
| Resolution power: | 8 cm$^{-1}$ |
| Time resolution power: | 0.2 sec |

In measurement, it was confirmed, by determining that the intensity of a benzene ring (near 1485 cm$^{-1}$) derived from a polysulfone resin was 0.1 or more, that the ATR prism was in contact with the sample.
Note that the time point when the OH peak was saturated refers to the time point when an increase of the intensity of the peak (3000 to 3700 cm$^{-1}$) derived from a hydroxy group was no longer observed compared to the peak intensity of the benzene ring of the polysulfone resin (near 1485 cm$^{-1}$).

(2) Determination of time point when the content of water reaches saturation point

The obtained spectrum data was normalized based on the intensity of benzene ring (near 1485cm$^{-1}$) derived from a polysulfone resin. A base line was set between 2700 cm$^{-1}$ and 3800 cm$^{-1}$ and the peak area of the hydroxy group was calculated.
Smoothing processing was carried out by averaging time-series integrated intensity data of a hydroxy-group (data of 9 points in total including 4-points before and after hydroxy-group peak). After the smoothing processing, an average of the slopes (an increase rate) of 10 data points (10 data points: the data point obtained by smoothing processing plus 9 data points obtained above) was obtained and the point at which the average slope showed 0 or less was determined as the time point when the content of water reaches a saturation point.
Note that, after the time point when the slope reached 0, if an increase (5% or more) of area was observed among 30 points (6s), it was determined that the content of water did not reach saturation, and the following data was further analyzed and the point satisfying the above conditions was determined as the time point when the content of water reached a saturation point.

(3) Data analysis by chemometrics

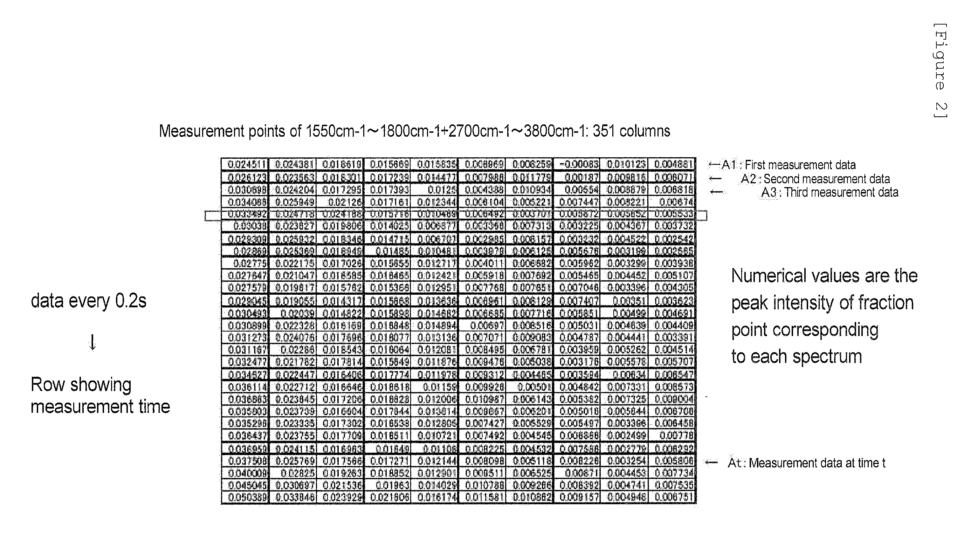

Data was analyzed based on one of chemometrics called alternating least square (ALS) method using the following software and calculator.
Software used in calculation: Mathworks (Natick, MA) MATLAB ver. R2008a
Calculator: Fujitsu FMV LIFEBOOK
Specific procedures will be described below.
Measurement was performed every 0.2 seconds to obtain a spectrum. From the spectrum, the intensity value per wavelength (intervals of 3.858 cm$^{-1}$, 351 points in total) was measured in the ranges of 1550 to 1800 cm$^{-1}$ and 2700 to 3800 cm$^{-1}$. The obtained data were stored in rows; whereas the spectra measured every 0.2 seconds were stored in columns. In this way, experiment spectrum matrix A was prepared. An example of the matrix is shown in Figure 2.
Next, spectrum matrix A was decomposed into 4 components (pure spectrum matrix K), which consists of 3 chemical components, that is, non-freezing water (hereinafter, referred to as "bound water" since a hydrogen binding region was optically detected in this Example), intermediate water and free water, and a differential spectrum, and concentration matrix C corresponding each of them. At this time, restriction was imposed on the matrix in order to uniquely perform the decomposition. More specifically, spectral resolution was performed by imposing a non-negative condition: a pure spectrum and concentration matrix never have negative elements in the ALS method. This is based on the ground that an absorption spectrum and concentration never take negative values. In the calculation for obtaining a compromise solution, if a negative value appeared, the negative value was forcibly replaced with zero and then regression calculation was repeated. In this way, all matrix elements were converged so as to satisfy the non-negative condition to obtain spectral solutions, C and K.

$$A = CK \qquad \text{expression (1)}$$

Provided that a first measurement spectrum is represented by $A_1$; a second measurement spectrum by $A_2$; ⋯ a spectrum of time t after initiation of measurement by At; spectra of bound water, intermediate water and free water, and differential spectrum are represented by $K_1$, $K_2$, $K_3$, and $K_4$ (at this time point, it is not known as to which one of $K_1$, $K_2$, $K_3$, and $K_4$ corresponds to which component); concentration ratio of 4 components at the time point t after initiation of measurement are represented by $C_{1t}$, $C_{2t}$, $C_{3t}$, and $C_{4t}$, expression (1) can be expressed by the following expression (2).
[Expression 1]

$$\begin{bmatrix} A_1 \\ A_2 \\ \cdot \\ \cdot \\ A_t \\ \cdot \\ \cdot \end{bmatrix} = \begin{bmatrix} C_{11} & C_{21} & C_{31} & C_{41} \\ C_{12} & C_{22} & C_{32} & C_{42} \\ & & \cdot & \\ & & \cdot & \\ & & \cdot & \\ C_{1t} & C_{2t} & C_{3t} & C_{4t} \end{bmatrix} \begin{bmatrix} K_1 \\ K_2 \\ K_3 \\ K_4 \end{bmatrix} \qquad \text{Expression (2)}$$

Spectra $K_1$, $K_2$, $K_3$, $K_4$ were obtained by generating random numbers in matrix C shown in Expression (2) and replacing a negative value with zero based on the non-negative condition that a concentration value never takes a negative value. Subsequently, C was obtained by replacing a component having a negative value in spectrum K with 0, based on the non-negative condition that a spectrum never takes a negative value. Furthermore, K was obtained from C based on the non-negative condition. This operation was repeated until all components of K and C reached 0 or more to obtain a solution.
From the obtained K and C, a spectrum where concentration values are close to zero was determined as a differential spectrum. Of the other 3 spectra, a spectrum having a large peak in the range of 1550 to 1800 $cm^{-1}$ and no peaks in the range of 3100 to 3500 $cm^{-1}$ was determined as the spectrum of bound water; a spectrum having a peak near 3400 $cm^{-1}$ as the spectrum of intermediate water, and a spectrum having peaks (wide peak) at 3200 and 3400 $cm^{-1}$ as the spectrum of free water.
From $C_{1t}$, $C_{2t}$, $C_{3t}$, $C_{4t}$ (30 measurement points) in the period of 6 seconds after the content of water reached a saturation point, the value of the differential spectrum was subtracted and again concentration ratio was calculated to obtain the average concentration values of bound water, intermediate water and free water. In this manner, the proportion of intermediate water was obtained.

[Measurement of water content]

The water content of a separation membrane was obtained as follows. A blood treatment device was decomposed without applying a preprocessing such as priming and the hollow separation membrane (about 1 g) was taken as a sample and accurately weighed. Thereafter, the sample was subjected to vacuum dry at 60°C for 12 hr. and then weighed to obtain a weight loss. The weight loss was regarded as the water content of the separation content and the water content of the separation membrane was calculated. Provided that the weight before drying is represented by $W_1$ and the weight after drying by $W_2$, the water content of the separation membrane W (%) is represented by the following expression (3).
[Expression 2]

$$W(\%) = \frac{W_1 - W_2}{W_1} \times 100 \qquad \text{Expression (3)}$$

[Measurement of weight average molecular weight]

To the polymer to be measured, an eluent was added so as to obtain a polymer concentration of 1.0 mg/mL to prepare a solution mixture. The solution mixture was stirred at room temperature, allowed to stand still overnight to dissolve the polymer and filtered by a 0.45 micron filter. The obtained filtrate was used as a sample. A synthetic polymer such as polyhydroxyalkyl methacrylate was measured by gel permeation chromatography in the following conditions:

Data processing: Tosoh Corp, GPC-8020
Apparatus: Tosoh Corp, HLC-8220GPC
Column: Two columns of TSK gel Super AWM-H (6.0 mm ID × 15 cm)
Oven: 40°C
Eluent: 5 mmol/L LiBr in DMF (0.6 mL/min)
Sample amount: 4 0 μL × 1.0 mg/mL
Detector: RI (Refractive Index Detector)
Calibration standard polymer: polystyrene (EasiCal (PS-1) manufactured by Agilent)

[Measurement of solubility to water (100 g) at 20°C]

The solubility of a synthetic polymer such as polyhydroxyalkyl methacrylate to water (100 g) at 20°C was measured as follows. Water (100 g) was placed together with a rotator in a flask. While controlling the temperature of a thermostatic chamber at 20°C, the polymer (5 g) to be measured was added and stirred for 12 hours or more. Thereafter, the mixture was filtered by use of a filter paper (No. 5A) and the insoluble matter on the filter paper was sufficiently washed with water and dried together with the filter paper at 60°C until a constant value was obtained. The weight of the insoluble matter was measured and difference between input (5 g) and the weight of the insoluble component was obtained and regarded as the solubility to water (100 g).

[Measurement of viscosity of a 0.1% aqueous solution at 25°C]

The viscosity of a 0.1% aqueous solution of a polymer derived from a natural substance such as a sodium salt of a polysaccharide at 25°C, was determined as follows. The polymer to be measured (0.5 g) was placed in water (500 g). The mixture was stirred for 12 hours or more while controlling the temperature at 25°C in a thermostatic chamber. The viscosity of the mixture was measured by a viscometer, Viscotester: VT-03F No. 4 rotor manufactured by RION.

[Measurement of concentration of polymer in the functional separation surface of the separation membrane]

The separation membrane was taken from a blood treatment device and cut along the fiber axis to expose the functional separation surface of the separation membrane. The concentration of the polymer in the functional separation surface of the separation membrane was determined by X-ray photoelectron spectroscopy in the following conditions.
Measurement apparatus: Thermo Fisher ESCALAB250
X-ray source: monochrome AlKα 15 kV × 10 mA
Analysis size: ellipse having a major axis of about 1 mm
Take off angle of photoelectron: 0° (spectroscope axis is placed in perpendicular to the sample surface)
Take-in region
Survey scan: 0 to 1,100 eV
Narrow scan: C1s, O1s, N1s, S2p, Na1s
Pass Energy
Survey scan: 100 eV
Narrow scan: 20 eV
The concentration of the polymer in the functional separation surface of the separation membrane was determined as follows. In the case of polyhydroxyalkyl methacrylate, since sulfur and nitrogen were not contained, the amounts of carbon and oxygen derived from a polysulfone resin were calculated from the amount of sulfur, and the amounts of carbon and oxygen derived from polyvinylpyrrolidone were calculated from the amount of nitrogen. The differences in carbon and oxygen amounts are regarded to be those derived from the polymer to obtain the concentration of the polymer on a weight basis. In the case of a sodium salt of a polysaccharide, the amount of polysaccharide was calculated from the amount of sodium. Depending upon the structure, the amounts of nitrogen and sulfur were obtained. Based on the difference in amounts of nitrogen and sulfur, the amounts of polyvinylpyrrolidone and polysulfone resin were calculated, respectively. Since hydrogen is not detected by X-ray photoelectron spectroscopy, hydrogen is excluded from calculation. The carbon amount, oxygen amount, nitrogen amount, sulfur amount and sodium amount were obtained as relative amounts (atomic%) from the integrated intensity values of C1s, O1s, N1s, S2p, Na1s by use of relative sensitivity factors (C1s: 1.00, O1s: 2.72, N1s: 1.68, S2p: 1.98, Na1s: 10.2) of individual elements.

The amount of polymer in the functional separation surface of the separation membrane was obtained based on the expression:

$$\text{(concentration by weight of polymer in the functional separation surface of the separation membrane)} / \text{(concentration by weight of polyvinylpyrrolidone in the functional separation surface of the separation membrane).}$$

[Measurement of lactate dehydrogenase (LDH) activity]

The blood compatibility of a separation membrane was evaluated based on the adherability of platelets to a membrane surface and quantified based on the activity of lactate dehydrogenase contained in the platelets attached to the membrane, as an index.

The inner surface of a hollow separation membrane was washed with the physiological saline (Otsuka saline, manufactured by Otsuka Pharmaceutical Co., Ltd.), at a rate of 100 mL/min per 1.5 m$^2$ for 5 minutes and thereafter the outer surface of the hollow separation membrane was washed at a rate of 500 mL/min for 2 minutes. In this way, priming was performed. After the priming, the blood treatment device was decomposed and the separation membrane was obtained. The both ends of the separation membrane were processed with an epoxy resin so as to have an effective length of 15 cm and a membrane inner surface area of 5 × 10$^{-3}$ m$^2$ to prepare a mini-module. To the mini-module, more specifically, to the inside of the hollow fiber, physiological saline (10 mL) was supplied to wash. Thereafter, heparin-added human blood (15 mL, heparin 1000 IU/L) was circulated at a flow rate of 1.3 mL/min through the mini-module prepared above at 37°C for 4 hours. The inside and the outside of the mini-module were separately washed with physiological saline (10 mL). From the mini-module washed, a half number of hollow fiber membranes having a length of 7 cm were taken, cut into pieces and placed in Spitz tubes for measuring LDH. These are used as samples.

Subsequently, a 0.5 vol% Triton X-100/PBS solution, which was prepared by dissolving Triton X-100 (Nacalai Tesque Inc.) in a phosphate buffer solution (PBS) (Wako Pure Chemical Industries Ltd.) was added to each of the Spitz tubes for LDH measurement in an amount of 0.5 mL and subjected to ultrasonic processing for 60 minutes. In this way, cells (mainly platelets) attached to the separation membrane were destroyed to extract LDH in the cells. An aliquot (0.05 mL) was taken from the extract. To this, a 0.6 mM sodium pyruvate solution (2.7 mL) and a 1.277 mg/mL nicotinamide adenine dinucleotide (NADH) solution (0.3 mL) was added and allowed to react. Immediately after the reaction, an aliquot of 0.5 mL was taken from the reaction solution and subjected to absorbance measurement at 340 nm. The remaining liquid was further reacted at 37°C for one hour and subjected to absorbance measurement at 340 nm. In this way, reduction of absorbance immediately after the reaction was obtained. With respect to the separation membrane (blank), which was not reacted with blood, the absorbance was measured in the same manner. Based on Expression (4) below, difference in absorbance was calculated. In this method, a larger degree of the reduction means that the LDH activity is high, more specifically, means that adhesion amount of platelets to the membrane surface is large. Note that, measurement was performed three times and an average value thereof was described.

$$\Delta_{340nm} = \text{(absorbance of sample immediately after reaction − absorbance of sample after 60 minutes) − (absorbance of blank immediately after reaction − absorbance of blank after 60 minutes)} \quad \text{expression (4)}$$

A separation membrane having excellent blood compatibility preferably has an LDH activity of 40 or less.

[Measurement of the concentration of nitric acid ion eluted from inner surface]

A blood treatment device was decomposed without applying a preprocessing such as priming and a hollow separation membrane was sampled. The both ends of the hollow separation membrane sample were processed with an epoxy resin so as to have an effective length of 15 cm and a membrane inner surface area of $12.2 \times 10^{-3}$ m$^2$ to prepare a mini-module. The mini-module was set such that distilled water (Otsuka Pharmaceutical Co., Ltd.) for injection of 37°C flowed downwardly at a flow rate of 3 mL/min. Distilled water was supplied inside hollow fibers. The solution that came out was sampled (5 ml) and put in a PS transparent tube (sterilized, code: 2-466-01, manufactured by AS ONE Corporation). The concentration of nitric acid ion in the solution was determined by ion chromatography in the conditions shown in Table 2.

[Table 2]
Measurement conditions of ion chromatography

| | |
|---|---|
| Apparatus: | Tosoh IC-2001 |
| Column: | TSKgel Super IC-AZ |
| Eluent: | 6.3 mM $NaHCO_3$ + 1.7 mM $Na_2CO_3$ |
| Flow rate: | 0.8 mL/min |
| Pressure: | 9.0 mPa |

According to Non Patent Literature 2, the no-observed-adverse-effect level (NOAEL) of nitrate nitrogen is 1.5 mg/kg body weight/day. This is oral intake. Assuming that a safety factor is 1000 times, if a person (body weight: 60 kg) undergoes dialysis three time per week, the acceptable amount per dialysis becomes 0.21 mg/session. If a blood purifier having a membrane area of 2.5 m$^2$ is used, the acceptable concentration of nitric acid ion in this measurement is calculated as 0.21 ppm.

[Example 1]

A spinning dope for membrane formation was prepared by dissolving 17 parts by mass of a polysulfone (P-1700 manufactured by Solvey) and 4 parts by mass of polyvinylpyrrolidone (K-90, manufactured by BASF) in 79 parts by mass of dimethylacetamide (special-grade reagent, manufactured by Kishida Chemical Co., Ltd.).
As a hollow-internal fluid, a 60 mass% aqueous dimethylacetamide solution was used.
The spinning dope for membrane formation and hollow-internal fluid were ejected from a tube-in-orifice type spinneret. The temperature of the spinning dope for membrane formation at the time of ejection was set at 40°C. The spinning dope for membrane formation was ejected, passed through a drop portion covered with a hood and soaked in a coagulation bath (60°C) consisting of water to coagulate. The spinning rate was set at 30 m/minute.
After the coagulation, the coagulants were washed with water and bundled such that the module to be fabricated had an effective membrane area of 1.5 m$^2$, wrapped in PE film, placed in a drying chamber and dried while supplying heated steam of 180°C to obtain a hollow separation membrane. The temperature of washing water was set at 90°C and the washing time was set at 180 seconds.
The ejection amounts of spinning dope for membrane formation and hollow-internal fluid were controlled so as to obtain a membrane having a thickness of 35 $\mu$m and an inner diameter of 185 $\mu$m after drying.
The obtained separation membrane was fabricated into a module having an effective membrane area of 1.5 m$^2$ and subjected to sterilization by an electron beam at 25 kGy under an air atmosphere to obtain a blood treatment device.
The proportion of intermediate water was 41% and the water content of the separation membrane was 2.9%. LDH activity was 25 [$\Delta$abs/hr/m$^2$] and the concentration of nitric acid ion eluted from the inner surface was 0.12 ppm. These results were satisfactory.

[Example 2]

A spinning dope for membrane formation was prepared by dissolving 17 parts by mass of a polysulfone (P-1700 manufactured by Solvey) and 4 parts by mass of polyvinylpyrrolidone (K-90, manufactured by BASF) in 79 parts by mass of dimethylacetamide (special-grade reagent, manufactured by Kishida Chemical Co., Ltd.).
The hollow-internal fluid was prepared by dissolving polyhydroxypropyl methacrylate (weight average molecular weight: 330,000, solubility: less than 0.1 g, manufactured by *Aldrich*) in a 60 mass% aqueous dimethylacetamide solution so as to obtain a concentration of 0.03 mass%.
The spinning dope for membrane formation and hollow-internal fluid were ejected from a tube-in-orifice type spinneret. The temperature of the spinning dope for membrane formation at the time of ejection was set at 40°C. The spinning dope for membrane formation was ejected, passed through a drop portion covered with a hood and soaked in a coagulation bath (60°C) consisting of water to coagulate. The spinning rate was set at 30 m/minute.

After the coagulation, washing with water and drying were performed to obtain a hollow separation membrane. The temperature of washing water was set at 90°C and the washing time was set at 180 seconds.

The ejection amounts of spinning dope for membrane formation and hollow-internal fluid were controlled so as to obtain a membrane having a thickness of 35 μm and an inner diameter of 185 μm after drying.

The obtained separation membrane was fabricated into a module having an effective membrane area of 1.5 $m^2$ and subjected to sterilization by an electron beam at 20 kGy under an air atmosphere to obtain a blood treatment device.

The proportion of intermediate water was 53%; the water content of the separation membrane was 0.6%; and the amount of polymer was 2.2 times as large as polyvinylpyrrolidone. LDH activity was 12 [Δabs/hr/m$^2$] and the concentration of nitric acid ion eluted from the inner surface was 0.08 ppm. These results were satisfactory.

[Example 3]

A hollow separation membrane was obtained in the same manner as in Example 2 except that the hollow-internal fluid, which was prepared by dissolving polyhydroxyethyl methacrylate (weight average molecular weight: 350,000, solubility: less than 0.1 g, manufactured by Scientific Polymer Products, Inc) in a 60 mass% aqueous dimethylacetamide solution so as to obtain a concentration of 0.1 mass%, was used.

The obtained separation membrane was fabricated into a module having an effective membrane area of 1.5 $m^2$ and subjected to sterilization by an electron beam at 50 kGy under an air atmosphere to obtain a blood treatment device.

The proportion of intermediate water was 45%; the water content of the separation membrane was 1.0%; the amount of polymer was 2.9 times as large as polyvinylpyrrolidone; the LDH activity was 17 [Δabs/hr/m$^2$] and the concentration of nitric acid ion eluted from the inner surface was 0.18 ppm. These results were satisfactory.

[Example 4]

A hollow separation membrane was obtained in the same manner as in Example 2 except that the hollow-internal fluid, which was prepared by dissolving polyhydroxypropyl methacrylate (weight average molecular weight: 330,000, solubility: less than 0.1 g, manufactured by Aldrich) in a 60 mass% aqueous dimethylacetamide solution so as to obtain a concentration of 0.01 mass%, was used.

The obtained separation membrane was fabricated into a module having an effective membrane area of 1.5 $m^2$ and subjected to sterilization by gamma ray at 25 kGy under an air atmosphere to obtain a blood treatment device.

The proportion of intermediate water was 61%; the water content of the separation membrane was 0.8%; the amount of polymer was 1.3 times as large as polyvinylpyrrolidone; the LDH activity was 6 [Δabs/hr/m$^2$] and the concentration of nitric acid ion eluted from the inner surface was 0.07 ppm. These results were satisfactory.

[Example 5]

A hollow separation membrane was obtained in the same manner as in Example 2 except that a 60 mass% aqueous dimethylacetamide solution was used as the hollow-internal fluid.

The obtained separation membrane was fabricated into a module having an effective membrane area of 1.5 $m^2$.

A coating liquid was prepared by dissolving polyhydroxypropyl methacrylate (weight average molecular weight: 330,000, solubility: less than 0.1 g, manufactured by *Aldrich*) in a 60 mass% aqueous isopropyl alcohol solution so as to obtain a concentration of 0.05 mass%.

The coating liquid (100 mL) was injected through a header cap having a liquid introduction (ejection) nozzle at a rate of 200 mL/minute and then excessive solution was removed by compressed air.

Thereafter, vacuum dry was performed until a constant amount was obtained. After completion of drying, electron beam sterilization at 25 kGy was performed under an air atmosphere to obtain a blood treatment device.

The proportion of intermediate water was 48%; the water content of the separation membrane was 1.1%; the amount of polymer was 1.7 times as large as polyvinylpyrrolidone; and the LDH activity was 15 [Δabs/hr/m$^2$] and the concentration of nitric acid ion eluted from the inner surface was 0.09 ppm. The results were satisfactory.

[Example 6]

A hollow separation membrane was obtained in the same manner as in Example 2 except that a 60 mass% aqueous dimethylacetamide solution was used as the hollow-internal fluid.

The obtained separation membrane was fabricated into a module having an effective membrane area of 1.5 $m^2$.

The coating liquid was prepared by adding sodium alginate (viscosity of a 0.1% solution: 3 mPa·s, manufactured by KIMICA Corporation) in water so as to obtain a concentration of 0.006 mass%.

The coating liquid (100 mL) was injected through a header cap having a liquid introduction (ejection) nozzle at a rate of 200 mL/minute and then excessive solution was removed by compressed air.

Thereafter, vacuum dry was performed until a constant amount was obtained. After completion of drying, electron beam sterilization at 25 kGy was performed under an air atmosphere to obtain a blood treatment device.

The proportion of intermediate water was 40%; the water content of the separation membrane was 3.0%; the amount of polymer was 2.2 times as large as polyvinylpyrrolidone; and the LDH activity was 37 [$\Delta$abs/hr/m$^2$] and the concentration of nitric acid ion eluted from the inner surface was 0.15 ppm. The results were satisfactory.

[Example 7]

A hollow separation membrane was obtained in the same manner as in Example 2 except that a spinning dope for membrane formation, which was prepared by dissolving 17 parts by mass of a poly ether sulfone (SUMIKAEXCEL 4800P, manufactured by Sumitomo Chemical Co., Ltd) and 4 parts by mass of polyvinylpyrrolidone (K-90, manufactured by BASF) in 79 parts by mass of dimethylacetamide (special-grade reagent, manufactured by Kishida Chemical Co., Ltd.), was used.

The obtained separation membrane was fabricated into a module having an effective membrane area of 1.5 m$^2$ and subjected to sterilization by an electron beam at 25 kGy under an air atmosphere to obtain a blood treatment device.

The proportion of intermediate water was 42%; the water content of the separation membrane was 0.7%; the amount of polymer was 2.3 times as large as polyvinylpyrrolidone; the LDH activity was 19 [$\Delta$abs/hr/m$^2$] and the concentration of nitric acid ion eluted from the inner surface was 0.13 ppm. These results were satisfactory.

[Example 8]

A hollow separation membrane was obtained in the same manner as in Example 2 except that the hollow-internal fluid, which was prepared by dissolving polyhydroxyethyl methacrylate (weight average molecular weight: 505,000, solubility: less than 0.1 g, manufactured by Scientific Polymer Products, Inc) in a 60 mass% aqueous dimethylacetamide solution so as to obtain a concentration of 0.2 mass%, was used.

The obtained separation membrane was fabricated into a module having an effective membrane area of 1.5 m$^2$ and subjected to sterilization by gamma ray at 25 kGy under an air atmosphere to obtain a blood treatment device.

The proportion of intermediate water was 55%; the water content of the separation membrane was 1.3%; the amount of polymer was 2.2 times as large as polyvinylpyrrolidone; the LDH activity was 8 [$\Delta$abs/hr/m$^2$] and the concentration of nitric acid ion eluted from the inner surface was 0.08 ppm. These results were satisfactory.

[Example 9]

A hollow separation membrane was obtained in the same manner as in Example 2 except that the hollow-internal fluid, which was prepared by dissolving polyhydroxyethyl methacrylate (weight average molecular weight: 1,300,000, solubility: less than 0.1 g, manufactured by Scientific Polymer Products, Inc) in a 60 mass% aqueous dimethylacetamide solution so as to obtain a concentration of 0.2 mass%, was used.

The obtained separation membrane was fabricated into a module having an effective membrane area of 1.5 m$^2$ and subjected to sterilization by gamma ray at 25 kGy under an air atmosphere to obtain a blood treatment device.

The proportion of intermediate water was 52%; the water content of the separation membrane was 1.5%; the amount of polymer was 2.1 times as large as polyvinylpyrrolidone; the LDH activity was 9 [$\Delta$abs/hr/m$^2$] and the concentration of nitric acid ion eluted from the inner surface was 0.11 ppm. These results were satisfactory.

[Comparative Example 1]

A blood treatment device was obtained in the same manner as in Example 2 except that a 60 mass% aqueous dimethylacetamide solution was used as the hollow-internal fluid.

The proportion of intermediate water was 12% and the water content of the separation membrane was 0.7%. In Comparative Example 1, since a polymer having a function of suppressing radiation deterioration of polyvinylpyrrolidone was not contained in the hollow-internal fluid and such a polymer was not used in coating by using a coating liquid, the amount of intermediate water in the functional separation surface of the separation membrane was low; LDH activity was 398 [$\Delta$abs/hr/m$^2$]; and unfavorable platelet adhesion was observed. In addition, the concentration of nitric acid ion eluted from the inner surface was as large as 0.33 ppm.

[Comparative Example 2]

A blood treatment device was obtained in the same manner as in Example 2 except that a spinning dope for membrane formation, which consisted of 17 parts by mass of a polysulfone (P-1700 manufactured by Solvey) and 83 parts by mass of dimethylacetamide (special-grade reagent, manufactured by Kishida Chemical Co., Ltd.), and a hollow-internal fluid, which was prepared by dissolving polyhydroxypropyl methacrylate (weight average molecular weight: 330,000, solubility: less than 0.1 g, manufactured by *Aldrich*) in a 60 mass% aqueous dimethylacetamide solution so as to obtain a concentration of 0.1 mass%, were used.
The proportion of intermediate water was 1.4%; and the water content of the separation membrane was 0.5%. In Comparative Example 2, since polyvinylpyrrolidone was not contained in the spinning dope for membrane formation; LDH activity was 393 [$\Delta$abs/hr/m$^2$]; and unfavorable platelet adhesion was observed. However, since polyvinylpyrrolidone was not contained, elution of nitric acid ion from the inner surface was not detected.

[Comparative Example 3]

A blood treatment device was obtained in the same manner as in Example 2 except that the electron beam irradiation dose was set at 100 kGy.
The proportion of intermediate water was 15%; the water content of the separation membrane was 0.6%; and the amount of polymer was 2.2 times as large as polyvinylpyrrolidone. The LDH activity was 72 [$\Delta$abs/hr/m$^2$] ; and platelet adhesion was observed. In addition, the concentration of nitric acid ion eluted from the inner surface was as large as 0.45 ppm.

[Comparative Example 4]

A blood treatment device was obtained in the same manner as in Example 6 except that the coating liquid, which was prepared by dissolving glycerin in water so as to obtain a concentration of 1 mass%, was used.
The proportion of intermediate water was 4.9%; the water content of the separation membrane was 11%; the LDH activity was 398 [$\Delta$abs/hr/m$^2$]; and unfavorable platelet adhesion was observed. However, elution of nitric acid ion was not detected because of elution of a large amount of glycerin.

[Comparative Example 5]

A blood treatment device was obtained in the same manner as in Example 2 except that a hollow-internal fluid, which was prepared by dissolving polyhydroxyethyl acrylate (weight average molecular weight: 260,000, manufactured by Scientific Polymer Products, Inc.) in a 60 mass% aqueous dimethylacetamide solution so as to obtain a concentration of 0.1 mass% was used as the hollow-internal fluid and the electron beam irradiation dose was set at 25 kGy.
The proportion of intermediate water was 13%; the water content of the separation membrane was 0.9%; and the amount of polymer was 1.3 times as large as polyvinylpyrrolidone. In Comparative Example 5, since polyhydroxyethyl acrylate does not have a function of suppressing radiation deterioration of polyvinylpyrrolidone, the amount of intermediate water in the functional separation surface of the separation membrane was low; and LDH activity was 375 [$\Delta$abs/hr/m$^2$]; and unfavorable platelet adhesion was observed. In addition, the concentration of nitric acid ion eluted from the inner surface was as large as 0.31 ppm.

[Comparative Example 6]

As the polymer, poly(vinylpyrrolidone-vinyl acetate) (manufactured by Wako Pure Chemical Industries Ltd.) was used. The solubility to water (100 g) thereof was 2.1 g and the weight average molecular weight thereof was 51,000.
A blood treatment device was obtained in the same manner as in Example 3 except that a hollow-internal fluid prepared by dissolving poly(vinylpyrrolidone-vinyl acetate) in a 60 mass% aqueous dimethylacetamide solution so as to obtain a concentration of 0.01 mass%.
The proportion of intermediate water was 15%; the water content of the separation membrane was 1.1%; and the amount of polymer was 0.9 times. The amount of intermediate water in the functional separation surface of the separation membrane was low; and LDH activity was 135 [$\Delta$abs/hr/m$^2$]; and adhesion of platelets was observed. In addition, the concentration of nitric acid ion eluted from the inner surface was 0.25 ppm.

[Comparative Example 7]

A blood treatment device was obtained in the same manner as in Example 2 except that the concentration of polyhydroxypropyl methacrylate in the hollow-internal fluid was 0.003%.

The proportion of intermediate water was 18%; the water content of the separation membrane was 0.6%; and the amount of polymer was 0.3 times as large as polyvinylpyrrolidone. The LDH activity was 65 [$\Delta$abs/hr/m$^2$]; and adhesion of platelets was observed. In addition, the concentration of nitric acid ion eluted from the inner surface was as large as 0.22 ppm.

[Table 3]

| | Example 1 | Example 2 | Example 3 | Example 4 | Example 5 | Example 6 | Example 7 | Example 8 | Example 9 |
|---|---|---|---|---|---|---|---|---|---|
| Polysulfone resin | PS | PS | PS | PS | PS | PS | PES | PS | PS |
| Polyvinylpyrrolidone | ○ | ○ | ○ | ○ | ○ | ○ | ○ | ○ | ○ |
| Drying | heated steam | Hot air | Hot air | Hot air | Hot air | Hot air | Hot air | Hot air | Hot air |
| Polymer | None | Polyhydroxypropyl methacrylate PHPMA | Polyhydroxyethyl methacrylate PHEMA | Polyhydroxypropyl methacrylate PHPMA | Polyhydroxypropyl methacrylate PHPMA | Sodium Alginate | Polyhydroxypropyl methacrylate PHPMA | Polyhydroxyethyl methacrylate PHEMA | Polyhydroxyethyl methacrylate PHEMA |
| Weight average molecular weight | - | 330,000 | 350,000 | 330,000 | 330,000 | - | 330,000 | 505,000 | 1,300,000 |
| Viscosity (0.1%) | - | - | - | - | - | 3mPa·s | - | - | - |
| Ratio of intermediate water/% | 41 | 53 | 45 | 61 | 48 | 40 | 42 | 55 | 52 |
| Water content/% | 2.9 | 0.6 | 1.0 | 0.8 | 1.1 | 3.0 | 0.7 | 1.3 | 1.5 |
| Concentration of nitric acid ion eluted from the inner surface/ppm | 0.12 | 0.08 | 0.18 | 0.07 | 0.09 | 0.15 | 0,13 | 0.08 | 0.11 |
| Addition site | None | Hollow-internal fluid | Hollow-internal fluid | Hollow-internal fluid | Coating liquid | Coating liquid | Hollow-internal fluid | Hollow-internal fluid | Hollow-internal fluid |
| Addition concentration | None | 0.03 mass% (300 ppm) | 0.1 mass% (1000 ppm) | 0.01 mass% (100 ppm) | 0.05 mass% (500 ppm) | 0.006 mass% (60 ppm) | 0.03 mass% (300 ppm) | 0.02 mass% (200 ppm) | 0.02 mass% (200 ppm) |
| Sterilization | EB-25kGy | EB-20kGy | EB-50kGy | γ-25kGy | EB-25kGy | EB-25kGy | EB-25kGy | γ-25kGy | γ-25kGy |
| LDH activity* | 25 | 12 | 17 | 6 | 15 | 37 | 19 | 8 | 9 |
| Polymer/PVP | 0 | 2.2 | 2.9 | 1.3 | 1.7 | 2.2 | 2.3 | 2.2 | 2.1 |
| *unit:△abs/hr/m$^2$ | | | | | | | | | |

[Table 4]

| | Comparative Example 1 | Comparative Example 2 | Comparative Example 3 | Comparative Example 4 | Comparative Example 5 | Comparative Example 6 | Comparative Example 7 |
|---|---|---|---|---|---|---|---|
| Polysulfone resin | PS | PS | PS | PS | PS | PS | PS |
| Polyvinylpyrrolidone | ○ | - | ○ | ○ | ○ | ○ | ○ |
| Drying | Hot air | Hot air | Hot air | Hot air | Hot air | Hot air | Hot air |
| Polymer | - | Polyhydroxypropyl methacrylate PHPMA | Polyhydroxypropyl methacrylate PHPMA | Glycerin | Polyhydroxyethyl acrylate PHEA | Poly(vinylpyrrolidone-vinyl acetate) | Polyhydroxypropyl methacrylate PHPMA |
| Weight average molecular weight | - | 330,000 | 330,000 | 92 | 260,000 | 51,000 | 330,000 |
| Viscosity | - | - | - | - | - | *Solubility to water 2.1 g | - |
| Ratio of intermediate water/% | 12 | 1.4 | 15 | 4.9 | 13 | 15 | 18 |
| Water content/% | 0.7 | 0.5 | 0.6 | 11 | 0.9 | 1.1 | 0.6 |
| Concentration of nitric acid ion eluted from the inner surface/ppm | 0.33 | N.D. | 0.45 | Not detected | 0.31 | 0.25 | 0.22 |
| Addition site | - | Hollow-internal fluid | Hollow-internal fluid | Coating liquid | Hollow-internal fluid | Hollow-internal fluid | Hollow-internal fluid |
| Addition concentration | - | 0.1 mass% (1000 ppm) | 0.03 mass% (300 ppm) | 1 mass% | 0.1 mass% (1000 ppm) | 0.01 mass% (100 ppm) | 0.003 mass% (30 ppm) |
| Sterilization | EB-20kGy | EB-20kGy | EB-100kGy | EB-25kGy | EB-25kGy | EB-50kGy | EB-20kGy |
| LDH activity* | 398 | 393 | 72 | 398 | 375 | 135 | 65 |
| Polymer/PVP | 0 | No PVP | 2.2 | 0 | 1.3 | 0.9 | 0.3 |
| *unit:$\Delta$abs/hr/m$^2$ | | | | | | | |

The present application is based on Japanese Patent Application No. 2012-132275 filed on June 11, 2012, the content of which is incorporated herein by reference.

Industrial Applicability

The separation membrane for blood treatment and the blood treatment device of the present invention having the membrane incorporated therein exhibits satisfactory blood compatibility and an effect of reducing the amount of nitric acid ion eluted from the inner surface even if it is subjected to radiation sterilization in dry state and provided as dry-state separation membrane for blood treatment, and can be suitably used for an extracorporeal circulation therapy such as hemodialysis, hemofiltration, hemodiafiltration, blood component fractionation, supplying oxygen and plasma separation.

**Claims**

1. A separation membrane for blood treatment containing at least a polysulfone resin and polyvinylpyrrolidone, wherein a proportion of intermediate water present in water in a functional separation surface of the separation membrane is 40% or more at the time when the content of water reaches a saturation point by impregnating a dry-state separation membrane with water, a water content of the separation membrane is 10% or less, and the separation membrane is subjected to radiation sterilization.

2. The separation membrane for blood treatment according to Claim 1, wherein an amount of nitric acid ion eluted from an inner surface is 0.2 ppm or less.

3. The separation membrane for blood treatment according to Claim 1 or 2, having a polymer having a function of suppressing radiation deterioration of polyvinylpyrrolidone at least in the functional separation surface of the separation membrane.

4. The separation membrane for blood treatment according to any one of Claims 1 to 3, wherein the radiation sterilization is performed at an irradiation dose of 15 to 50 kGy.

5. The separation membrane for blood treatment according to Claim 3 or 4, wherein the polymer having a function of suppressing radiation deterioration of polyvinylpyrrolidone is at least one selected from the group consisting of polyhydroxyalkyl methacrylate and a sodium salt of a polysaccharide.

6. A blood treatment device containing the separation membrane for blood treatment according to any one of Claims 1 to 5 incorporated therein.

7. A method for producing a separation membrane for blood treatment containing at least a polysulfone resin and polyvinylpyrrolidone, comprising the steps of:

forming a separation membrane containing at least a polysulfone resin and polyvinylpyrrolidone,
drying the never dry membrane with heated steam up to a water content of the separation membrane of 10% or less, and
subjecting the separation membrane to radiation sterilization.

8. A method for producing a separation membrane for blood treatment containing at least a polysulfone resin and polyvinylpyrrolidone, comprising the step of:

forming a separation membrane containing at least a polysulfone resin and polyvinylpyrrolidone,
drying the separation membrane up to a water content of 10% or less, and
subjecting the separation membrane, which has a polymer having a function of suppressing radiation deterioration of polyvinylpyrrolidone in the functional separation surface of the separation membrane, to radiation sterilization.

9. The production method according to Claim 8, comprising a step of coating a functional separation surface of the separation membrane with the polymer by ejecting a spinning dope for membrane formation containing at least a polysulfone resin and polyvinylpyrrolidone and a hollow-internal fluid containing the polymer, followed by spinning.

**10.** The production method according to Claim 8, comprising a step of coating the functional separation surface of the separation membrane with the polymer by injecting a coating liquid containing the polymer into the separation membrane containing at least a polysulfone resin and polyvinylpyrrolidone.

[Figure 1]

Micro-syringe

Filter paper

Dropping of water

Hollow fiber

ATR prism measurement position

Functional separation surface of hollow-fiber separation membrane

Filter paper

IR

ATR prism

[Figure 2]

Measurement points of 1550cm-1~1800cm-1+2700cm-1~3800cm-1: 351 columns

data every 0.2s

Row showing measurement time

A1 : First measurement data
A2 : Second measurement data
A3 : Third measurement data

Numerical values are the peak intensity of fraction point corresponding to each spectrum

At : Measurement data at time t

## INTERNATIONAL SEARCH REPORT

International application No.

PCT/JP2013/066053

### A. CLASSIFICATION OF SUBJECT MATTER

*A61M1/18*(2006.01)i, *B01D69/02*(2006.01)i, *B01D69/08*(2006.01)i, *B01D69/12* (2006.01)i, *B01D71/08*(2006.01)i, *B01D71/38*(2006.01)i, *B01D71/44* (2006.01)i, *B01D71/68*(2006.01)i, *D01D5/24*(2006.01)i, *A61K35/14*(2006.01)n,

According to International Patent Classification (IPC) or to both national classification and IPC

### B. FIELDS SEARCHED

Minimum documentation searched (classification system followed by classification symbols)
A61M1/18, B01D69/02, B01D69/08, B01D69/12, B01D71/08, B01D71/38, B01D71/44, B01D71/68, D01D5/24, A61K35/14, A61P7/08

Documentation searched other than minimum documentation to the extent that such documents are included in the fields searched

Jitsuyo Shinan Koho 1922–1996 Jitsuyo Shinan Toroku Koho 1996–2013
Kokai Jitsuyo Shinan Koho 1971–2013 Toroku Jitsuyo Shinan Koho 1994–2013

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X<br>Y | JP 2005-87945 A (Toray Industries, Inc.),<br>07 April 2005 (07.04.2005),<br>paragraphs [0015], [0027]<br>(Family: none) | 7<br>1-2,4,6 |
| X<br>Y | JP 2010-233980 A (Asahi Kasei Kuraray Medical Co., Ltd.),<br>21 October 2010 (21.10.2010),<br>paragraphs [0055] to [0058], [0069] to [0070]<br>(Family: none) | 8,10<br>1-6,9 |
| Y | JP 2006-288942 A (Toyobo Co., Ltd.),<br>26 October 2006 (26.10.2006),<br>claim 8; paragraphs [0037], [0082], [0102] to [0106]<br>(Family: none) | 1-6 |

☒ Further documents are listed in the continuation of Box C. ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 10 September, 2013 (10.09.13) | 17 September, 2013 (17.09.13) |

| Name and mailing address of the ISA/<br>Japanese Patent Office | Authorized officer |
|---|---|
| Facsimile No. | Telephone No. |

Form PCT/ISA/210 (second sheet) (July 2009)

27

**INTERNATIONAL SEARCH REPORT**

International application No.

PCT/JP2013/066053

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2000-254222 A (Terumo Corp.), 19 September 2000 (19.09.2000), paragraph [0045] (Family: none) | 1-6 |
| Y | WO 2006/104117 A1 (Toray Industries, Inc.), 05 October 2006 (05.10.2006), paragraph [0024] & US 2009/0242477 A1 paragraph [0036] & JP 2012-254350 A     & EP 1894965 A1 & CN 101151303 A     & KR 10-2007-0118148 A | 1-6 |
| Y | JP 9-66225 A (Terumo Corp.), 11 March 1997 (11.03.1997), claim 1; paragraph [0042] & EP 749775 A1 page 5, lines 49 to 51 | 9 |
| Y | WO 2002/09857 A1 (Asahi Medical Co., Ltd., NOF Corp.), 07 February 2002 (07.02.2002), page 9, lines 28 to 30; page 11, lines 17 to 21 & US 2004/0045897 A1 paragraphs [0048], [0039] & EP 1306121 A1 | 9 |
| P,X | WO 2013/015046 A1 (Asahi Kasei Medical Co., Ltd.), 31 January 2013 (31.01.2013), paragraphs [0036], [0063] (Family: none) | 1-6,8,10 |
| P,X | WO 2013/065819 A1 (Asahi Kasei Medical Co., Ltd.), 10 May 2013 (10.05.2013), paragraphs [0010], [0019] to [0020], [0036], [0046] to [0058] (Family: none) | 1-6,8,9 |
| A | JP 11-332980 A (Toray Industries, Inc.), 07 December 1999 (07.12.1999), entire text; all drawings (Family: none) | 1-7 |

Form PCT/ISA/210 (continuation of second sheet) (July 2009)

**INTERNATIONAL SEARCH REPORT**

International application No.

PCT/JP2013/066053

Continuation of A. CLASSIFICATION OF SUBJECT MATTER
  (International Patent Classification (IPC))

*A61P7/08*(2006.01)n

        (According to International Patent Classification (IPC) or to both national
        classification and IPC)

Form PCT/ISA/210 (extra sheet) (July 2009)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- JP 3551971 B
- JP 2002212333 A
- JP 6296686 A
- JP 4338223 A
- JP 7194949 A
- JP 2006016575 A
- JP 2012132275 A

## Non-patent literature cited in the description

- **KEN TANAKA.** The Roles of organized water molecules in the biointerface, Sakigake Live 2004. *Nano-technology field Joint Research Debrief Session, Summary of lecture in the section of "Systemization and Function,* 2005, 24-33, http://jstore.jst.go.jp/PDFView.html?type=research&i d=3090&property=researchReportPdfList&index=0
- Soft Drink Evaluation Document, nitrate nitrogen/nitrite nitrogen (draft), Food Safety Commission. *chemical substance/contaminant specialist research meeting,* May 2012